(19) Europäisches Patentamt
European Patent Office
Office européen des brevets

(11) **EP 3 438 246 B1**

(12) **EUROPEAN PATENT SPECIFICATION**

(45) Date of publication and mention
of the grant of the patent:
**26.08.2020 Bulletin 2020/35**

(21) Application number: **17774880.3**

(22) Date of filing: **27.03.2017**

(51) Int Cl.:
*C12N 5/071* (2010.01)        *A61K 9/70* (2006.01)
*A61K 35/34* (2015.01)        *A61K 47/42* (2017.01)
*A61L 27/22* (2006.01)        *A61L 27/38* (2006.01)
*A61L 27/44* (2006.01)        *A61P 9/00* (2006.01)
*C07K 14/78* (2006.01)        *A61L 27/36* (2006.01)

(86) International application number:
**PCT/JP2017/012285**

(87) International publication number:
**WO 2017/170343 (05.10.2017 Gazette 2017/40)**

(54) **LAMINATE CONTAINING CELL SHEET, AGENT FOR TREATING CARDIAC DISEASES, AND FILM FOR BEING LAMINATED ON CELL SHEET**

LAMINAT MIT ZELLLAGE, WIRKSTOFF FÜR DIE BEHANDLUNG VON HERZERKRANKUNGEN UND FILM ZUM LAMINIEREN DER ZELLLAGE

STRATIFIÉ CONTENANT UNE COUCHE CELLULAIRE, AGENT THÉRAPEUTIQUE POUR LE TRAITEMENT DES MALADIES CARDIAQUES ET FILM STRATIFIÉ SUR LA COUCHE CELLULAIRE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **29.03.2016 JP 2016065395**

(43) Date of publication of application:
**06.02.2019 Bulletin 2019/06**

(73) Proprietors:
• **FUJIFILM Corporation**
  **Tokyo 106-8620 (JP)**
• **Terumo Kabushiki Kaisha**
  **Tokyo 151-0072 (JP)**

(72) Inventors:
• **NAKAMURA, Kentaro**
  **Ashigarakami-gun**
  **Kanagawa 258-8577 (JP)**
• **SANO, Shinya**
  **Ashigarakami-gun**
  **Kanagawa 259-0151 (JP)**
• **KURUMA, Yosuke**
  **Ashigarakami-gun**
  **Kanagawa 259-0151 (JP)**
• **SAMESHIMA, Tadashi**
  **Ashigarakami-gun**
  **Kanagawa 259-0151 (JP)**

(74) Representative: **Klunker IP**
**Patentanwälte PartG mbB**
**Destouchesstraße 68**
**80796 München (DE)**

(56) References cited:
**EP-A1- 2 940 126          EP-A1- 3 006 559**
**WO-A1-2008/103041      WO-A1-2014/104366**
**WO-A1-2014/192909      WO-A2-2006/107207**
**JP-A- 2008 538 707       JP-A- 2010 519 251**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

SEQUENCE LISTING

<110> FUJIFILM Corporation
<120> A cell sheet-containing laminate, an agent for therapy of heart disease, and a film for lamination on cell sheet
<130>\201@16F02151

<160> 10

<170> PatentIn version 3.5

<210> 1

<211> 571

<212> PRT

<213> Recombinant

<400> 1

```
Gly Ala Pro Gly Ala Pro Gly Leu Gln Gly Ala Pro Gly Leu Gln Gly
1               5                   10                  15
Met Pro Gly Glu Arg Gly Ala Ala Gly Leu Pro Gly Pro Lys Gly Glu
            20                  25                  30
Arg Gly Asp Ala Gly Pro Lys Gly Ala Asp Gly Ala Pro Gly Ala Pro
            35                  40                  45
Gly Leu Gln Gly Met Pro Gly Glu Arg Gly Ala Ala Gly Leu Pro Gly
        50                  55                  60
Pro Lys Gly Glu Arg Gly Asp Ala Gly Pro Lys Gly Ala Asp Gly Ala
65                  70                  75                  80
Pro Gly Lys Asp Gly Val Arg Gly Leu Ala Gly Pro Ile Gly Pro Pro
                85                  90                  95
Gly Glu Arg Gly Ala Ala Gly Leu Pro Gly Pro Lys Gly Glu Arg Gly
            100                 105                 110
Asp Ala Gly Pro Lys Gly Ala Asp Gly Ala Pro Gly Lys Asp Gly Val
            115                 120                 125
Arg Gly Leu Ala Gly Pro Ile Gly Pro Pro Gly Pro Ala Gly Ala Pro
            130                 135                 140
Gly Ala Pro Gly Leu Gln Gly Met Pro Gly Glu Arg Gly Ala Ala Gly
145                 150                 155                 160
Leu Pro Gly Pro Lys Gly Glu Arg Gly Asp Ala Gly Pro Lys Gly Ala
                165                 170                 175
Asp Gly Ala Pro Gly Lys Asp Gly Val Arg Gly Leu Ala Gly Pro Pro
                180                 185                 190
Gly Ala Pro Gly Leu Gln Gly Ala Pro Gly Leu Gln Gly Met Pro Gly
                195                 200                 205
Glu Arg Gly Ala Ala Gly Leu Pro Gly Pro Lys Gly Glu Arg Gly Asp
            210                 215                 220
Ala Gly Pro Lys Gly Ala Asp Gly Ala Pro Gly Ala Pro Gly Leu Gln
225                 230                 235                 240
Gly Met Pro Gly Glu Arg Gly Ala Ala Gly Leu Pro Gly Pro Lys Gly
                245                 250                 255
Glu Arg Gly Asp Ala Gly Pro Lys Gly Ala Asp Gly Ala Pro Gly Lys
                260                 265                 270
Asp Gly Val Arg Gly Leu Ala Gly Pro Ile Gly Pro Pro Gly Glu Arg
            275                 280                 285
Gly Ala Ala Gly Leu Pro Gly Pro Lys Gly Glu Arg Gly Asp Ala Gly
            290                 295                 300
Pro Lys Gly Ala Asp Gly Ala Pro Gly Lys Asp Gly Val Arg Gly Leu
305                 310                 315                 320
Ala Gly Pro Ile Gly Pro Pro Gly Pro Ala Gly Ala Pro Gly Ala Pro
                325                 330                 335
Gly Leu Gln Gly Met Pro Gly Glu Arg Gly Ala Ala Gly Leu Pro Gly
            340                 345                 350
Pro Lys Gly Glu Arg Gly Asp Ala Gly Pro Lys Gly Ala Asp Gly Ala
            355                 360                 365
Pro Gly Lys Asp Gly Val Arg Gly Leu Ala Gly Pro Pro Gly Ala Pro
            370                 375                 380
Gly Leu Gln Gly Ala Pro Gly Leu Gln Gly Met Pro Gly Glu Arg Gly
385                 390                 395                 400
Ala Ala Gly Leu Pro Gly Pro Lys Gly Glu Arg Gly Asp Ala Gly Pro
```

```
                        405                    410                    415
        Lys Gly Ala Asp Gly Ala Pro Gly Ala Pro Gly Leu Gln Gly Met Pro
                    420                    425                    430
        Gly Glu Arg Gly Ala Ala Gly Leu Pro Gly Pro Lys Gly Glu Arg Gly
                    435                    440                    445
        Asp Ala Gly Pro Lys Gly Ala Asp Gly Ala Pro Gly Lys Asp Gly Val
                450                    455                    460
        Arg Gly Leu Ala Gly Pro Ile Gly Pro Pro Gly Glu Arg Gly Ala Ala
        465                    470                    475                    480
        Gly Leu Pro Gly Pro Lys Gly Glu Arg Gly Asp Ala Gly Pro Lys Gly
                    485                    490                    495
        Ala Asp Gly Ala Pro Gly Lys Asp Gly Val Arg Gly Leu Ala Gly Pro
                    500                    505                    510
        Ile Gly Pro Pro Gly Pro Ala Gly Ala Pro Gly Ala Pro Gly Leu Gln
                    515                    520                    525
        Gly Met Pro Gly Glu Arg Gly Ala Ala Gly Leu Pro Gly Pro Lys Gly
                530                    535                    540
        Glu Arg Gly Asp Ala Gly Pro Lys Gly Ala Asp Gly Ala Pro Gly Lys
        545                    550                    555                    560
        Asp Gly Val Arg Gly Leu Ala Gly Pro Pro Gly
                            565                    570
```

<210> 2
<211> 4
<212> PRT
<213> Artificial Sequence
<220>
<223> Description of Artificial Sequence: adhesive sequence
<400> 2

```
                        Arg Glu Asp Val
                        1
```

<210> 3
<211> 5
<212> PRT
<213> Artificial Sequence
<220>
<223> Description of Artificial Sequence: adhesive sequence
<400> 3

```
                        Tyr Ile Gly Ser Arg
                        1               5
```

<210> 4
<211> 5
<212> PRT
<213> Artificial Sequence
<220>
<223> Description of Artificial Sequence: adhesive sequence
<400> 4

```
                        Pro Asp Ser Gly Arg
                        1               5
```

<210> 5
<211> 7
<212> PRT
<213> Artificial Sequence

<220>
<223> Description of Artificial Sequence: adhesive sequence
<400> 5

```
Arg Tyr Val Val Leu Pro Arg
1               5
```

<210> 6
<211> 6
<212> PRT
<213> Artificial Sequence
<220>
<223> Description of Artificial Sequence: adhesive sequence
<400> 6

```
Leu Gly Thr Ile Pro Gly
1               5
```

<210> 7
<211> 10
<212> PRT
<213> Artificial Sequence
<220>
<223> Description of Artificial Sequence: adhesive sequence
<400> 7

```
Arg Asn Ile Ala Glu Ile Ile Lys Asp Ile
1               5                   10
```

<210> 8
<211> 5
<212> PRT
<213> Artificial Sequence
<220>
<223> Description of Artificial Sequence: adhesive sequence
<400> 8

```
Ile Lys Val Ala Val
1               5
```

<210> 9
<211> 4
<212> PRT
<213> Artificial Sequence
<220>
<223> Description of Artificial Sequence: adhesive sequence
<400> 9

```
Asp Gly Glu Ala
1
```

<210> 10
<211> 4
<212> PRT
<213> Artificial Sequence
<220>
<223> Description of Artificial Sequence: adhesive sequence
<400> 10

Glu Arg Gly Asp
1

**Description**

**BACKGROUND OF THE INVENTION**

1. Field of the Invention

The present invention relates to a laminate constituted with a cell sheet and a polymer film. The present invention also relates to an agent for treating cardiac diseases including the laminate. Furthermore, the present invention relates to a film for being laminated on a cell sheet.

2. Description of the Related Art

In recent years, regenerative medicine and cell transplantation therapy have been frequently practiced. Particularly, a therapy has been frequently examined in which cells are made into a cell sheet and only the cells are transplanted in the form of a sheet. It is considered that compared to the administration of individual cells as a suspension, the therapy results in better engraftment in the body and further improves the efficacy because the cell sheet is maintained as a structure. Patent Literature 1 aims to provide a manufacturing method using a cell culture solution, which does not contain impurity components resulting from the manufacturing process that hinder the clinical application, and describes a method for manufacturing a cell sheet including culturing cells having a density enabling the formation of a cell sheet substantially without causing growth in a cell culture solution containing no growth factor in an effective amount.

Meanwhile, regarding individual cells not being in the form of a sheet, a method is being examined in which the cells are transplanted by being combined with a scaffolding material that becomes a support of the cells. As the scaffolding material that becomes the support of the cells, gene recombinant gelatin is known. Patent Literature 2 describes gene recombinant gelatin which is useful for several uses accompanying cell adhesion such as a cell culture operation and accompanying the culture of scaffold-dependent cells, and is particularly useful for various medical uses.

Prior Art Literatures

Patent Literatures

   Patent Literature 1: JP2010-81829A
   Patent Literature 2: WO2008/103041A

EP 3 006 559 A1 and WO 2014/192909 A1 disclose a layered cell sheet incorporating hydrogel. The hydrogel is preferably in the form of particles.

EP 2 940 126 A1 and WO 2014/104366 A1 describe a corneal endothelial cell sheet formed on a support comprising a gelatin sheet. The gelatin sheet has a dry film thickness of from 5 to 40 $\mu$m.

WO 2006/107207 A2 discloses gelatin comprising films for cultivating cells. The films are formed at ambient conditions.

**SUMMARY OF THE INVENTION**

In a case where a sheet is prepared using only cells, unfortunately, the cell sheet is extremely brittle, and hence the original shape is not easily maintained, or it is difficult to handle the cell sheet. For transplanting the cell sheet, the above problems need to be solved. An object of the present invention is to provide a laminate containing a cell sheet which has hardness and is easy to handle at the time of transplantation or transport. Another object of the present invention is to provide a laminate for use in treating cardiac diseases.

In order to achieve the above objects, the inventors of the present invention conducted intensive examinations. As a result, the inventors have found that by laminating a biocompatible polymer film having a density of 500 $\mu$g/cm$^2$ to 10 mg/cm$^2$ on a cell sheet, the hardness and the handleability of the cell sheet can be improved, and have accomplished the present invention. According to the present invention, the following inventions are provided.

   [1] A laminate comprising a biocompatible polymer film having a density of 500 $\mu$g/cm$^2$ to 10 mg/cm$^2$, and a cell sheet disposed on at least one surface of the biocompatible polymer film, wherein the cell is a myocardial cell or a skeletal myoblast; and wherein
   the biocompatible polymer film satisfies the following Formula 1.

Formula 1: (swollen film thickness/dry film thickness) × 100 ≥ -27.5 × dry film thickness + 880

The unit of the swollen film thickness and the dry film thickness is μm.
[2] The laminate described in [1], in which the biocompatible polymer film satisfies the following Formula 2.

Formula 2: (swollen film thickness/dry film thickness) × 100 ≥ -27.5 × dry film thickness + 962.5

The unit of the swollen film thickness and the dry film thickness is μm.
[3] The laminate described in [1] or [2], in which a swelling ratio of the biocompatible polymer film represented by the following Formula 3 is equal to or higher than 230%.

Formula 3: (swollen film thickness/dry film thickness) × 100

The unit of the swollen film thickness and the dry film thickness is μm).
[4] The laminate described in any one of [1] to [3], in which the dry film thickness of the biocompatible polymer film is 5 to 200 μm.
[5] The laminate described in any one of [1] to [4], in which a wet film thickness of the biocompatible polymer film is 50 to 500 μm.
[6] The laminate described in any one of [1] to [5], in which the biocompatible polymer is recombinant gelatin.
[7] The laminate described in [6], in which the recombinant gelatin is represented by the following Formula 4.

Formula 4:　　　A-[(Gly-X-Y)$_n$]$_m$-B

In the formula, A represents any amino acid or any amino acid sequence, B represents any amino acid or any amino acid sequence, n X's each independently represent any amino acid, n Y's each independently represent any amino acid, n represents an integer of 3 to 100, m represents an integer of 2 to 10, and n sequences represented by Gly-X-Y may be the same as or different from each other.
[8] The laminate described in [6] or [7], in which the recombinant gelatin is represented by the following Formula 5.

Formula 5:　　　Gly-Ala-Pro-[(Gly-X-Y)$_{63}$]$_3$-Gly

In the formula, 63 X's each independently represent any amino acid, 63 Y's each independently represent any amino acid, and 63 sequences represented by Gly-X-Y may be the same as or different from each other.
[9] The laminate described in any one of [6] to [8], in which the recombinant gelatin has (1) amino acid sequence described in SEQ ID NO: 1 or (2) amino acid sequence which shares a sequence identity equal to or higher than 80% with the amino acid sequence described in SEQ ID NO: 1 and has Incompatibility.
[10] The laminate described in any one of [6] to [9], in which the recombinant gelatin has the amino acid sequence described in SEQ ID NO: 1.
[11] The laminate described in any one of [1] to [10] for use in treating cardiac diseases.

According to the laminate of the present invention, the hardness and the handleability of a cell sheet can be improved. According to the laminate for use in treating cardiac diseases of the present invention, cardiac diseases can be treated. The film for being laminated on a cell sheet of the present invention is useful in manufacturing the laminate of the present invention.

**BRIEF DESCRIPTION OF THE DRAWINGS**

Fig. 1 shows the results of evaluation of handleability of polymer films.
Fig. 2 shows a schematic view of a test system relating to a protein permeation test for a polymer film and the test results.

Fig. 3 shows the results of the protein permeation test for polymer films.

Fig. 4 shows the results obtained by plotting the protein permeability of the polymer films on a graph of a swelling ratio and a dry film thickness.

Fig. 5 shows how the fractional area change (FAC) of the left ventricular cavity changes after the transplantation to infarction models.

Fig. 6 shows the results obtained by checking skeletal myoblasts by immunostaining of a myosin heavy chain (MHC).

**DESCRIPTION OF THE PREFERRED EMBODIMENTS**

Hereinafter, embodiments of the present invention will be specifically described.

<Laminate>

The laminate of the present invention includes a biocompatible polymer film having a density of 500 $\mu$g/cm$^2$) to 10 mg/cm$^2$ and a cell sheet disposed on at least one surface of the biocompatible polymer film. In the present specification, the biocompatible polymer film is also referred to as a polymer film.

Specific examples of the laminate of the present invention include, but are not particularly limited to, a laminate including one sheet of biocompatible polymer film and one cell sheet (that is, a laminate in which a cell sheet is present only on one surface of a biocompatible polymer film) and a laminate including two cell sheets and one sheet of biocompatible polymer film between the cell sheets (that is, a laminate in which cell sheets are present on both surfaces of a biocompatible polymer film).

[Biocompatible polymer film]

(Characteristics of biocompatible polymer film)

The density of the biocompatible polymer film used in the present invention is 500 $\mu$g/cm$^2$ to 10 mg/cm$^2$. In a case where the density is within the above range, a laminate having sufficient hardness can be manufactured. The density of the biocompatible polymer film is preferably 500 $\mu$g/cm$^2$ to 5.0 mg/cm$^2$, and more preferably 500 $\mu$g/cm$^2$ to 2.0 mg/cm$^2$.

The density of the biocompatible polymer film is calculated by "coating mass/coating area" at the time of preparation. The coating mass means the mass of the biocompatible polymer applied.

It is preferable that the biocompatible polymer film used in the present invention satisfies the following Formula 1.

$$\text{Formula 1: (swollen film thickness/dry film thickness)} \times 100 \geq -27.5 \times \text{dry film thickness} + 880$$

The unit of the swollen film thickness and the dry film thickness is $\mu$m.

It is preferable that Formula 1 is satisfied, because then the handleability of the biocompatible polymer film is further improved. In a case where the film is prepared by drying a biocompatible polymer solution by allowing the solution to dry in a gelled state at 4°C, a biocompatible polymer film satisfying Formula 1 is obtained, and the handleability is further improved. In contrast, in a case where the biocompatible polymer film is prepared by drying the solution at room temperature (25°C), a biocompatible polymer film which does not satisfy Formula 1 is obtained.

It is more preferable that the biocompatible polymer film used in the present invention satisfies the following Formula 2.

$$\text{Formula 2: (swollen film thickness/dry film thickness)} \times 100 \geq -27.5 \times \text{dry film thickness} + 962.5$$

The unit of the swollen film thickness and the dry film thickness is $\mu$m.

In a case where Formula 2 is satisfied, the handleability of the biocompatible polymer film is further improved than in a case where Formula 1 is satisfied.

The dry film thickness is obtained by measuring the thickness of the dried biocompatible polymer film by using a micrometer (SOFT TOUCH MICRO CLM manufactured by Mitutoyo Corporation, and the like). The swollen film thickness is obtained by measuring the thickness of the biocompatible polymer film sufficiently wet with water for injection by using a micrometer.

The swelling ratio, represented by the following Formula 3, of the biocompatible polymer film used in the present invention is preferably equal to or higher than 230%.

$$\text{Formula 3: (swollen film thickness/dry film thickness)} \times 100$$

The unit of the swollen film thickness and the dry film thickness is $\mu$m.

In a case where the swelling ratio represented by Formula 3 is equal to or higher than 230%, the permeability of a protein (a 66 kDa protein in examples) can be improved. That is, it is preferable that the swelling ratio is equal to or higher than 230%, because then both the prevention of cell infiltration and the permeation of a protein (nutrient component or the like) can be achieved.

The swelling ratio represented by Formula 3 is more preferably equal to or higher than 250%, even more preferably equal to or higher than 270%, and particularly preferably equal to or higher than 300%. The upper limit of the swelling ratio represented by Formula 3 is not particularly limited, but is generally equal to or lower than 1,000%.

The dry film thickness of the biocompatible polymer film is not particularly limited, but is preferably 5 to 200 $\mu$m, more preferably 10 to 100 $\mu$m, even more preferably 20 to 50 $\mu$m, and particularly preferably 20 to 40 $\mu$m.

The wet film thickness of the biocompatible polymer film is not particularly limited, but is preferably 50 to 500 $\mu$m. In a case where the wet film thickness is within the above range, the warping of the biocompatible polymer film can be prevented. The wet film thickness of the biocompatible polymer film is more preferably 50 to 300 $\mu$m, and even more preferably 100 to 300 $\mu$m.

(Biocompatible polymer)

The biocompatibility means a property of not inducing a markedly harmful response such as a prolonged and chronic inflammatory response by contact with a biological body. It does not matter whether or not the biocompatible polymer used in the present invention is decomposed in a biological body as long as the biocompatible polymer exhibits biocompatibility in a biological body, but the biocompatible polymer is preferably a biodegradable polymer. Specific examples of non-biodegradable polymers include polytetrafluoroethylene (PTFE), polyurethane, polypropylene, polyester, vinyl chloride, polycarbonate, acryl, silicone, 2-methacryloyloxyethyl phosphorylcholine (MPC), and the like. Specific examples of biodegradable polymers include a polypeptide (for example, gelatin which will be described later) such as a naturally occurring peptide, a recombinant peptide, or a chemically synthesized peptide, polylactic acid, polyglycolic acid, a lactic acid·glycolic acid copolymer (PLGA), hyaluronic acid, glycosamineglycan, proteoglycan, chondroitin, cellulose, agarose, carboxymethyl cellulose, chitin, chitosan, and the like. Among these, a recombinant peptide is particularly preferable. These biocompatible polymers may be treated so as to improve the cell adhesiveness thereof. Specifically, it is possible to use methods such as "coating a substrate surface with a cell adhesion substrate (fibronectin, vitronectin, or laminin) or a cell adhesion sequence (an RGD sequence, an LDV sequence, an REDV sequence, a YIGSR sequence, a PDSGR sequence, an RYVVLPR sequence, an LGTIPG sequence, an RNIAEIIKDI sequence, an IKVAV sequence, an LRE sequence, a DGEA sequence, and an HAV sequence represented by one-letter notation for amino acids) peptide", "amination or cationization of a substrate surface", or "a hydrophilic treatment on a substrate surface by a plasma treatment or corona discharge".

The type of the polypeptide including the recombinant peptide or the chemically synthesized peptide is not particularly limited as long as the polypeptide has biocompatibility. As the polypeptide, gelatin, collagen, atelocollagen, elastin, fibronectin, pronectin, laminin, tenascin, fibrin, fibroin, entactin, thrombospondin, and retronectin are preferable, and gelatin, collagen, and atelocollagen are most preferable. As the gelatin to be used in the present invention, natural gelatin, recombinant gelatin, or chemically synthesized gelatin is preferable, and recombinant gelatin is more preferable. The natural gelatin means gelatin made from naturally occurring collagen.

The chemically synthesized peptide or the chemically synthesized gelatin means a peptide or gelatin that is artificially synthesized. The synthesis of a peptide such as gelatin may be solid-phase synthesis or liquid-phase synthesis, and is preferably solid-phase synthesis. The solid-phase synthesis of a peptide is known to those skilled in the related art, and examples thereof include an Fmoc group synthesis method in which a Fluorenyl-Methoxy-Carbonyl group (Fmoc group) is used for protecting an amino group, a Boc group synthesis method in which a tert-Butyl Oxy Carbonyl group (Boc group) is used for protecting an amino group, and the like. Details of the recombinant gelatin that will be described later in the present specification can be applied to the preferred aspect of the chemically synthesized gelatin.

"1/IOB" value which is a hydrophilicity value of the biocompatible polymer used in the present invention is preferably 0 to 1.0, more preferably 0 to 0.6, and even more preferably 0 to 0.4. IOB is an index of hydropathicity based on the organic conception diagram showing polarity/non-polarity of organic compounds that was suggested by Atsushi Fujita. Details of IOB are described, for example, in "Pharmaceutical Bulletin", vol. 2, 2, pp. 163-173 (1954), "Scope of Chemistry", vol. 11, 10, pp.719-725 (1957), "Fragrance Journal", vol. 50, pp. 79-82 (1981), and the like. In brief, methane (CH4) is regarded as the origin of all organic compounds, all other compounds are regarded as derivatives of methane, and a certain numerical value is assigned to the number of carbon atoms, the substituent, the transformative portion, the ring, and the like of the compounds. The scores are added up so as to determine an organic value (OV) and an inorganic value (IV), and a graph is created by plotting the organic value on the X-axis and the inorganic value on the Y-axis. IOB in the organic conception diagram refers to a ratio of the inorganic value (IV) to the organic value (OV) in the organic conception diagram, that is, "inorganic value (IV)/organic value (OV)". For details of the organic conception diagram, see "New Edition of Organic Conception Diagram-Fundamentals and Applications-" (Yoshiki Koda et al., SANKYO SHUPPAN Co., Ltd., 2008). In the present specification, hydropathicity is represented by "1/IOB" value which is the reciprocal of IOB showing that the smaller the "1/IOB" value (the closer the "1/IOB" value to 0), the higher the hydrophilicity.

In a case where the "1/IOB" value of the polymer used in the present invention is within the above range, the hydrophilicity is high, and the water absorbing properties are improved.

In a case where the biocompatible polymer used in the present invention is a polypeptide, a hydropathicity index thereof represented by a Grand average of hydropathicity (GRAVY) value is preferably equal to or lower than 0.3 and equal to or higher than -9.0, and more preferably equal to or lower than 0.0 and equal to or higher than -7.0. The Grand average of hydropathicity (GRAVY) value can be obtained by the methods in "Gasteiger E., Hoogland C., Gattiker A., Duvaud S., Wilkins M.R., Appel R.D., Bairoch A.; Protein Identification and Analysis Tools on the ExPASy Server;(In) John M. Walker (ed): The Proteomics Protocols Handbook, Humana Press (2005). pp. 571-607" and "Gasteiger E., Gattiker A., Hoogland C., Ivanyi I., Appel R.D., Bairoch A.; ExPASy: the proteomics server for in-depth protein knowledge and analysis.; Nucleic Acids Res. 31:3784-3788(2003)".

In a case where the GRAVY value of the polymer used in the present invention is within the above range, the hydrophilicity is high, and the water absorbing properties are improved.

(Recombinant gelatin)

The biocompatible polymer is preferably recombinant gelatin.

The recombinant gelatin is a polypeptide or a protein-like substance which is prepared by a gene recombination technology and has an amino acid sequence analogous to gelatin. The recombinant gelatin is preferably a gene recombinant having an amino acid sequence derived from a partial amino acid sequence of collagen.

It is preferable that the recombinant gelatin has a repeating sequence represented by Gly-X-Y (X and Y each independently represent any amino acid) characteristic of collagen. A plurality of sequences represented by Gly-X-Y may be the same as or different from each other.

As the recombinant gelatin, for example, it is possible to use those described in EP1014176, US6992172B, WO2004/85473A, WO2008/103041A, and the like. However, the recombinant gelatin is not limited thereto. As the recombinant gelatin used in the present invention, recombinant gelatin of the following aspect is preferable.

The recombinant gelatin exhibits excellent biocompatibility due to the performance inherent to the natural gelatin, does not carry a risk of causing bovine spongiform encephalopathy (BSE) because the recombinant gelatin is not derived from nature, and is excellently noninfectious. Furthermore, because the recombinant gelatin is more homogenous than the natural gelatin and has a predetermined sequence, it is possible to accurately design the recombinant gelatin while reducing the variation in strength and decomposition properties by crosslinking and the like.

The molecular weight of the recombinant gelatin is not particularly limited, but is preferably equal to or greater than 2,000 and equal to or smaller than 100,000 (equal to or greater than 2 kDa (kilodaltons) and equal to or smaller than 100 kDa), more preferably equal to or greater than 2,500 and equal to or smaller than 95,000 (equal to or greater than 2.5 kDa and equal to or smaller than 95 kDa), even more preferably equal to or greater than 5,000 and equal to or smaller than 90,000 (equal to or greater than 5 kDa and equal to or smaller than 90 kDa), and most preferably equal to or greater than 10,000 and equal to or smaller than 90,000 (equal to or greater than 10 kDa and equal to or smaller than 90 kDa).

It is preferable that the recombinant gelatin has a repeating sequence represented by Gly-X-Y characteristic of collagen. A plurality of sequences represented by Gly-X-Y may be the same as or different from each other. In Gly-X-Y, Gly represents glycine, and each of X and Y represents any amino acid (preferably any amino acid other than glycine). The sequence represented by Gly-X-Y characteristic of collagen is a partial structure extremely unique in the composition and sequence of the amino acid of gelatin-collagen compared to other proteins. Glycine takes up about 1/3 of this portion and is one of the three repeating amino acids in the amino acid sequence. Glycine is the simplest amino acid, and the disposition of the molecular chain thereof is restricted less. Furthermore, glycine makes a big contribution to the regeneration of a helix structure at the time of gelation. The amino acids represented by X and Y contain a large amount of imino acids (proline and oxyproline), and the content of the imino acids is preferably 10% to 45% of the total content of the amino acids. In the sequence of the recombinant gelatin, the proportion of amino acids constituted with the repeating structure of Gly-X-Y is preferably equal to or higher than 80%, more preferably equal to or higher than 95%, and most preferably equal to or higher than 99%.

Generally, gelatin contains charged polar amino acids and uncharged polar amino acids at 1:1. The polar amino acids specifically refer to cysteine, aspartic acid, glutamic acid, histidine, lysine, asparagine, glutamine, serine, threonine, tyrosine, and arginine. Among these, uncharged polar amino acids refer to cysteine, asparagine, glutamine, serine, threonine, and tyrosine. In the recombinant gelatin used in the present invention, the proportion of the polar amino acids in all the constituent amino acids is 10% to 40% and preferably 20% to 30%. The proportion of the uncharged amino acids in the polar amino acids is preferably equal to or higher than 5% and less than 20%, and more preferably equal to or higher than 5% and less than 10%. Furthermore, it is preferable that gelatin does not contain one amino acid and preferably does not contain two or more amino acids among serine, threonine, asparagine, tyrosine, and cysteine in the sequence thereof.

Generally, regarding polypeptides, minimal amino acid sequence functioning as cell adhesion signals are known (for example, "Pathophysiology" published from Nagai Publishing Co., Ltd., Vol. 9, No. 7 (1990), p. 527). The recombinant gelatin used in the present invention may have two or more cell adhesion signals in one molecule. Specifically, as such sequences, an RGD sequence, an LDV sequence, an REDV sequence, a YIGSR sequence, a PDSGR sequence, an RYVVLPR sequence, an LGTIPG sequence, an RNIAEIIKDI sequence, an IKVAV sequence, an LRE sequence, a DGEA sequence, and an HAV sequence represented by one-letter notation for amino acids are preferable, because these sequences enable the adhesion of many kinds of cells. Among these, an RGD sequence, a YIGSR sequence, a PDSGR sequence, an LGTIPG sequence, an IKVAV sequence, and an HAV sequence are more preferable, and an RGD sequence is particularly preferable. Among RGD sequences, an ERGD sequence is preferable.

Regarding the disposition of the RGD sequence in the recombinant gelatin used in the present invention, it is preferable that the number of amino acids between RGD sequences varies in a range of 0 to 100 and preferably varies in a range of 25 to 60.

The number of minimal amino acid sequences described above contained in one molecule of the protein is preferably 3 to 50, more preferably 4 to 30, particularly preferably 5 to 20, and most preferably 12.

In the recombinant gelatin used in the present invention, the ratio of the RGD motif to the total number of amino acids is preferably at least 0.4%. In a case where the recombinant gelatin contains 350 or more amino acids, it is preferable that each stretch of the 350 amino acids contains at least one RGD motif. The ratio of the RGD motif to the total number of amino acids is more preferably at least 0.6%, even more preferably at least 0.8%, still more preferably at least 1.0%, particularly preferably at least 1.2%, and most preferably at least 1.5%. The number of RGD motifs in the recombinant peptide is preferably at least 4, more preferably 6, even more preferably 8, and particularly preferably equal to or greater than 12 and equal to or smaller than 16 per 250 amino acids. The ratio of 0.4% of the RGD motif means that the recombinant gelatin contains at least one RGD sequence per 250 amino acids. The number of RGD motifs is an integer. Therefore, in order to satisfy the characteristic of 0.4%, gelatin constituted with 251 amino acids has to contain at least two RGD sequences. The recombinant gelatin of the present invention preferably contains at least two RGD sequences per 250 amino acids, more preferably contains at least three RGD sequences per 250 amino acids, and even more preferably contains at least four RGD sequences per 250 amino acids. In another aspect, the recombinant gelatin of the present invention contains at least four RGD motifs, preferably contains six RGD motifs, more preferably contains eight RGD motifs, and even more preferably contains twelve to sixteen RGD motifs.

The recombinant gelatin may be partially hydrolyzed.

It is preferable that the recombinant gelatin used in the present invention is represented by the following Formula 4.

Formula 4:          A-[(Gly-X-Y)$_n$]$_m$-B

In the formula, A represents any amino acid or any amino acid sequence, B represents any amino acid or any amino acid sequence, n X's each independently represent any amino acid, and n Y's each independently represent any amino acid. n is preferably an integer of 3 to 100, more preferably an integer of 15 to 70, and most preferably an integer of 50 to 65. m preferably represents an integer of 2 to 10, and more preferably represents an integer of 3 to 5. n sequences represented by Gly-X-Y may be the same as or different from each other.

It is more preferable that the recombinant gelatin used in the present invention is represented by the following Formula 5.

Formula 5:          Gly-Ala-Pro-[(Gly-X-Y)$_{63}$]$_3$-Gly

In the formula, 63 X's each independently represent any amino acid, 63 Y's each independently represent any amino acid, and 63 sequences represented by Gly-X-Y may be the same as or different from each other.

It is preferable that a plurality of sequence units of naturally occurring collagen are bonded to the repeating unit. The naturally occurring collagen is not limited as long as it exists in the nature. As the naturally occurring collagen, type I, type II, type III, type IV, or type V collagen is preferable, and type I, type II, or type III collagen is more preferable. According to another aspect, the collagen is preferably derived from human beings, cows, pigs, mice, or rats, and more preferably derived from human beings.

The isoelectric point of the recombinant gelatin used in the present invention is preferably 5 to 10, more preferably 6 to 10, and even more preferably 7 to 9.5. The isoelectric point of the recombinant gelatin can be determined by measuring pH after allowing a 1% by mass gelatin solution and cation and anion exchange resins to pass through a mixed crystal column as described in isoelectric focusing electrophoresis (see Maxey, C. R. (1976); Phitogr. Gelatin 2, Editor Cox, P. J. Academic, London, Engl.).

It is preferable that the recombinant gelatin is deaminated.

It is preferable that the recombinant gelatin does not have a telopeptide.

It is preferable that the recombinant gelatin is substantially a pure polypeptide prepared by nucleic acids encoding amino acid sequences.

It is particularly preferable that the recombinant gelatin has (1) amino acid sequence described in SEQ ID NO: 1 or (2) amino acid sequence which shares a sequence identity equal to or higher than 80% (preferably equal to or higher than 90%, more preferably equal to or higher than 95%, and particularly preferably equal to or higher than 98%) with the amino acid sequence described in SEQ ID NO: 1 and has biocompatibility.

It is most preferable that the recombinant gelatin has the amino acid sequence described in SEQ ID NO: 1.

In the present invention, the sequence identity refers to a value calculated by the following formula.

$$\% \text{ Sequence identity} = [(\text{number of same residues})/(\text{alignment length})] \times 100$$

The sequence identity shared between two amino acid sequences can be determined by any method known to those skilled in the related art by using a Basic Local Alignment Search Tool (BLAST) program (J. Mol. Biol. 215:403-410, 1990) and the like.

The recombinant gelatin may have an amino acid sequence which is obtained by the deletion, substitution, or addition of one or several amino acids in the amino acid sequence described in SEQ ID NO: 1 and has biocompatibility.

"One or several amino acids" in "amino acid sequence which is obtained by the deletion, substitution, or addition of one or several amino acids" preferably means 1 to 20 amino acids, more preferably means 1 to 10 amino acids, even more preferably means 1 to 5 amino acids, and particularly preferably means 1 to 3 amino acids.

The recombinant gelatin can be manufactured by gene recombination technologies known to those skilled in the related art. For example, the recombinant gelatin can be manufactured based on the methods described in EP1014176A2, US6992172B, WO2004/85473A, WO2008/103041A, and the like. Specifically, genes encoding an amino acid sequence of a predetermined recombinant gelatin are obtained and incorporated into an expression vector, thereby preparing a recombinant expression vector. The recombinant expression vector is introduced into an appropriate host, thereby preparing a transformant. By culturing the obtained transformant in an appropriate medium, recombinant gelatin is produced. Therefore, by collecting the recombinant gelatin produced from the culture, the recombinant gelatin used in the present invention can be prepared.

(Method for manufacturing biocompatible polymer film)

The method for manufacturing the biocompatible polymer film is not particularly limited. The biocompatible polymer film can be manufactured by common methods. For example, the biocompatible polymer film can be manufactured by pouring an aqueous solution of a biocompatible polymer into a plastic tray and drying the solution at a low temperature (for example, in a refrigerator at 4°C or the like) or room temperature. It is preferable that the aqueous solution of a biocompatible polymer is dried at a low temperature (for example, in a refrigerator at 4°C or the like).

The biocompatible polymer in the biocompatible polymer film can be crosslinked.

As general crosslinking methods, thermal crosslinking, crosslinking by aldehydes (for example, formaldehyde, glutaraldehyde, and the like), crosslinking by a condensation agent (carbodiimide, cyanamide, and the like), enzymatic crosslinking, optical crosslinking, ultraviolet crosslinking, hydrophobic interaction, hydrogen bonding, ionic interaction, and the like are known. In the present invention, these crosslinking methods can also be used. As the crosslinking method used in the present invention, thermal crosslinking, ultraviolet crosslinking, or enzymatic crosslinking is more preferable, and thermal crosslinking is particularly preferable.

In a case where the crosslinking is performed using an enzyme, the enzyme is not particularly limited as long as it functions to crosslink polymer materials with each other. The crosslinking can be performed preferably using transglutaminase and laccase, and most preferably using transglutaminase. Specific examples of proteins enzymatically crosslinked by transglutaminase are not particularly limited as long as they are proteins having a lysine residue and a glutamine residue. The transglutaminase may be derived from mammals or microorganisms. Specifically, examples thereof include an ACTIVA series manufactured by AJINOMOTO CO., INC., mammal-derived transglutaminase marketed as a reagent such as guinea pig liver-derived transglutaminase, goat-derived transglutaminase, and rabbit-derived transglutaminase manufactured by Oriental Yeast Co., ltd., Upstate USA Inc., Biodesign International, and the like, human-derived blood coagulation factors (Factor XIIIa, Haematologic Technologies, Inc.), and the like.

The reaction temperature at the time of performing the crosslinking (for example, thermal crosslinking) is not particularly limited as long as crosslinks can be formed. However, the reaction temperature is preferably -100°C to 500°C, more preferably 0°C to 300°C, even more preferably 50°C to 300°C, even more preferably 100°C to 250°C, and particularly preferably 120°C to 200°C.

The reaction time at the time of performing the crosslinking (for example, thermal crosslinking) is not particularly limited, but is generally 1 hour to 72 hours, preferably 2 hours to 48 hours, and more preferably 4 hours to 36 hours.

[Cell sheet]

In the present invention, the cell sheet means a sheet containing cells as a main component. The cell sheet is a substance in which cells form a sheet by being linked to each other. The constitution of the cell sheet is not particularly limited as long as the cell sheet has a sheet shape. The cell sheet may be any of a single-layer cell sheet, a sheet constituted with two or more layers formed of cells, and a sheet formed of three-dimensionally cultured cells.

The cells may be linked to each other directly and/or through an interpositional substance. The interpositional substance is not particularly limited as long as it is a substance which can link the cells to each other in at least a mechanical way, and examples thereof include the extracellular matrix and the like. The interpositional substance is preferably derived from cells and particularly derived from the cells constituting the cell sheet. The cells are linked to each other in at least a mechanical way, but the cells may also be linked to each other functionally, for example, chemically or electrically.

The cell sheet in the present invention contains any cells capable of forming a cell sheet. Examples of the cells are not particularly limited and include myocardial cells, myoblasts (for example, skeletal myoblasts), fibroblasts, synovial cells, epithelial cells, endothelial cells, and the like. Among these, myocardial cells and skeletal myoblasts are preferable. As the cells, it is possible to use cells derived from any organism which can be treated using the cell sheet. The organism is not particularly limited, and examples thereof include human beings, non-human primates (monkey and the like), dogs, cats, pigs, horses, goats, lambs, mice, rats, hamsters, and the like. One kind of cells may be used singly, or two or more kinds of cells may be used. In a case where a cell sheet is formed of two or more kinds of cells, a proportion (purity) of the most abundant cells is preferably equal to or higher than 25%, more preferably equal to or higher than 50%, and even more preferably equal to or higher than 60% at the end of the manufacturing of the cell sheet.

The cell sheet can be manufactured by known cell sheet manufacturing methods or methods equivalent thereto. For example, the cell sheet may be manufactured by culturing cells in a culture plate, allowing the cells to form a sheet, and then collecting the sheet from the culture plate. As described above, the method for manufacturing the cell sheet is not particularly limited, but for example, the cell sheet can be manufactured by the method described in JP2010-81829A. Specifically, the cell sheet may be manufactured by culturing cells, which have a density enabling the formation of a cell sheet substantially without causing growth, in a cell culture solution containing no growth factor in an effective amount.

"Density enabling the formation of a cell sheet substantially without causing growth" means that the cells have a density at which the cell sheet can be formed in a case where the cells are cultured in a culture solution containing no growth factor. For example, in the case of the skeletal myoblasts, in the method of the related art in which a culture solution containing a growth factor is used, in order to form a cell sheet, cells having a density of about 6,500 cells/cm$^2$ are seeded in a plate. However, even though the cells having the aforementioned density are cultured in a culture solution containing no growth factor, a cell sheet cannot be formed. For instance, for the skeletal myoblasts, "density enabling the formation of a cell sheet substantially without causing growth" is typically equal to or higher than 300,000 cells/cm$^2$. The upper limit of the cell density is not particularly limited as long as the formation of a cell sheet is not hindered and cell differentiation does not occur. For the skeletal myoblasts, the upper limit is 1,100,000 cells/cm$^2$, for example. Those skilled in the related art can appropriately determine an adequate cell density by experiments. The cells may or may not grow during the culture period, but even though the cells grow, the growth does not proceed to change the properties of the cells. For example, the skeletal myoblasts start to be differentiated after they become confluent. It is preferable that the skeletal myoblasts are seeded at a density at which a cell sheet can be formed but differentiation does not occur. It is preferable that the cells do not grow over the range of measurement error. Whether or not the cells have grown can be evaluated, for example, by comparing the number of cells at the time of seeding with the number of cells after the formation of a cell sheet. In the present aspect, the number of cells after the formation of a cell sheet is typically equal to or smaller than 300%, preferably equal to or smaller than 200%, more preferably equal to or smaller than 150%, even more preferably equal to or smaller than 125%, and particularly preferably equal to or smaller than 100% of the number of cells at the time of seeding.

For example, the cells are cultured for a predetermined period of time and preferably cultured for a period of time for which the cell differentiation does not occur. In this case, the cells remain undifferentiated during the culture period. Whether the cell differentiation has occurred can be evaluated by any method known to those skilled in the related art. For instance, in the case of the skeletal myoblasts, the expression of a myosin heavy chain (MHC) or the multinucleation of the cells can be used as an index of differentiation. The culture time is preferably equal to or shorter than 48 hours, more preferably equal to or shorter than 40 hours, and even more preferably equal to or shorter than 24 hours.

The growth factor means any substance which further stimulates the cell growth compared to cell growth without using the growth factor. Examples thereof include an epidermal growth factor (EGF), a vascular endothelial growth factor (VEGF), a fibroblast growth factor (FGF), and the like.

The effective amount of the growth factor means the amount of the growth factor which significantly stimulates the cell growth compared to cell growth without using the growth factor, or means, for the sake of convenience, the amount generally added for the purpose of causing cell growth in the technical field of the present invention. The significance of the stimulation of cell growth can be appropriately evaluated, for example, by any statistical technique known in the technical field of the present invention such as a t-test. Furthermore, the general addition amount can be ascertained from various known documents of the technical field of the present invention. Specifically, the effective amount of EGF in culturing the skeletal myoblasts is equal to or greater than 0.005 $\mu$g/mL, for example.

Accordingly, "containing no growth factor in an effective amount" means that the concentration of the growth factor in the culture solution is less than the effective amount. For example, during the culture of the skeletal myoblasts, the concentration of EGF in the culture solution is preferably less than 0.005 $\mu$g/mL, and more preferably less than 0.001 $\mu$g/mL. In a preferred aspect, the concentration of the growth factor in the culture solution is less than the general concentration thereof in a biological body. In such an aspect, for example, the concentration of EGF in the culture solution during the culture of the skeletal myoblasts is preferably less than 5.5 ng/mL, more preferably less than 1.3 ng/mL, and even more preferably less than 0.5 ng/mL. In a more preferred aspect, the culture solution in the present invention substantially does not contain a growth factor. Herein, "substantially does not contain" means that the content of the growth factor in the culture solution does not exert a negative influence in a case where the cell sheet is applied to a biological body, and preferably means that a growth factor is not added to the culture solution as far as possible. Therefore, in this aspect, the culture solution does not contain other components such as a growth factor at a concentration equal to or higher than, for example, the concentration of a growth factor contained in the serum or the like.

The cell culture solution (simply referred to as "culture solution" in some cases) is not particularly limited as long as it can keep cells survive. Typically, a culture solution containing amino acids, vitamins, and electrolytes as main components can be used. For example, the culture solution is based on a basal medium for cell culture. The basal medium is not limited, and examples thereof include the Dulbecco's modified Eagle's medium (DMEM), the Eagle's minimum essential medium (MEM), F12, Ham, RPMI 1640, MCDB (MCDB 102, 104, 107, 131, 153, 199, and the like), L15, SkBM (registered trademark), RITC 80-7, and the like. Many of these basal media are on the market, and the composition thereof is also known. The basal medium having the standard composition may be used as it is (for example, those on the market may be used as they are), or the composition may be appropriately modified according to the cell species or the cell conditions. Accordingly, the basal medium is not limited to those having known compositions, and those obtained by the addition, removal, increase, or reduction of one component or two or more components may also be used.

The amino acids contained in the basal medium are not particularly limited, and examples thereof include L-arginine, L-cysteine, L-glutamine, glycine, L-histidine, L-isoleucine, L-leucine, L-lysine, L-methionine, L-phenylalanine, L-serine, L-threonine, L-tryptophan, L-tyrosine, L-valine, and the like.

The vitamins contained in the basal medium are not particularly limited, and examples thereof include calcium D-pantothenate, choline chloride, folic acid, i-inositol, niacinamide, riboflavin, thiamine, pyridoxine, biotin, lipoic acid, vitamin B$_{12}$, adenine, thymidine, and the like.

The electrolytes contained in the basal medium are not particularly limited, and examples thereof include CaCl$_2$, KCl, MgSO$_4$, NaCl, NaH$_2$PO$_4$, NaHCO$_3$, Fe(NO$_3$)$_3$, FeSO$_4$, CuSO$_4$, MnSO$_4$, Na$_2$SiO$_3$, (NH$_4$)6Mo$_7$O$_{24}$, NaVO$_3$, NiCl$_2$, ZnSO$_4$, and the like.

The basal medium may contain, in addition to these components, saccharides such as D-glucose, sodium pyruvate, a pH indicator such as phenol red, putrescine, and the like.

In a case where the cell sheet is assumed to be applied to a human being, the cell culture solution substantially does not contain a steroid component. Herein, "steroid component" refers to, among the compounds having a steroid nucleus, compounds that can exert negative influences such as hypoadrenocorticism and Cushing's syndrome on a biological body. Such compounds are not particularly limited, and examples thereof include cortisol, prednisolone, triamcinolone, dexamethasone, betamethasone, and the like. Accordingly, "substantially does not contain a steroid component" means that the content of the aforementioned compounds in the culture solution does not exert a negative influence in a case where the cell sheet is applied to a biological body, and preferably means that those compounds are not added to the culture solution as far as possible, that is, the culture solution does not contain other components such as a steroid component at a concentration equal to or higher than, for example, the concentration of a steroid component contained in the serum or the like.

In a case where the cell sheet is assumed to be applied to a human being, the cell culture solution substantially does not contain a heterologous serum component. Herein, "heterologous serum component" means a serum component derived from an organism of a species different from that of a recipient. For example, in a case where a human being is a recipient, a serum derived from a cow or a horse such as fetal bovine serum (FBS, FCS), calf serum (CS), horse serum (HS), or the like corresponds to the heterologous serum component. Accordingly, "substantially does not contain a heterologous serum component" means that the content of these sera in the culture solution does not exert negative influences in a case where the cell sheet is applied to a biological body (for example, the content of serum albumin in the cell sheet is less than 50 ng), and preferably means that these substances are not added to the culture solution as far as possible.

In a case where the cell sheet is assumed to be applied to a human being, the cell culture solution contains a homologous serum component. Herein, "homologous serum component" means a serum component derived from an organism of the same species as that of a recipient. For example, in a case where a human being is a recipient, human serum corresponds to the homologous serum component. It is preferable that the homologous serum component is an autologous serum component, that is, a serum component derived from a recipient. The content of the homologous serum component is not particularly limited as long as it is an amount enabling the formation of a cell sheet, but is preferably 5 to 40 v/v (volume/volume)%, and more preferably 10 to 20 v/v (volume/volume)%.

In an example, the cell culture solution substantially does not contain a selenium component. Herein, "selenium component" includes a selenium molecule, a selenium-containing compound which is particularly a selenium-containing compound capable of releasing a selenium molecule in a biological body, such as selenious acid, and the like. Accordingly, "substantially does not contain a selenium component" means that the content of these substances in the culture solution does not exert negative influences in a case where the cell sheet is applied to a biological body, and preferably means that these substances are not added to the culture solution as far as possible, that is, the culture solution does not contain other components such as a selenium component at a concentration equal to or higher than, for example, the concentration of a selenium component contained in the serum or the like. Specifically, in the case of the human being, the selenium concentration in the culture solution is lower than a value obtained by multiplying the normal selenium concentration (for example, 10.6 to 17.4 $\mu$g/dL) in the human serum by the proportion of the human serum contained in the medium (that is, provided that the content of the human serum is 10%, the selenium concentration is 1.0 to 1.7 $\mu$g/dL, for example).

Typically, the cell sheet is manufactured by a step of seeding cells in a culture solution and a step of forming a cell sheet by culturing the cells.

The cell culture can be performed under the conditions that are generally adopted in the technical field of the present invention. For example, typically, the cells are cultured under the conditions of 37°C and 5% $CO_2$. From the viewpoint of sufficiently forming a cell sheet and preventing cell differentiation, the culture period is preferably equal to or shorter than 48 hours, more preferably equal to or shorter than 40 hours, and even more preferably equal to or shorter than 24 hours. The culture can be performed using a container of any size and shape.

<Method for manufacturing laminate>

The method for manufacturing the laminate of the present invention is not particularly limited as long as it is a method which makes it possible to laminate a cell sheet and a biocompatible polymer film. For example, the cell sheet and the biocompatible polymer film may be separately prepared, and then the biocompatible polymer film may be laminated on a surface of the cell sheet or the cell sheet may be laminated on a surface of the biocompatible polymer film. Alternatively, a biocompatible polymer film may be prepared in advance, and then cells may be cultured by being seeded on a surface of the biocompatible polymer film such that a cell sheet is formed, thereby preparing a laminate.

<Agent for treating cardiac diseases>

The laminate of the present invention can be used for treating diseases or injuries of a subject. For example, the cell sheet formed of skeletal myoblasts can be used as an agent for treating cardiac diseases. Examples of the cardiac diseases include myocardial infarction, ischemic cardiomyopathy, dilated cardiomyopathy, angina, and the like.

Examples of the method for administering the agent for treating cardiac diseases including the laminate of the present invention include a method of directly applying the agent for treating cardiac diseases to the affected region of a damaged myocardial tissue, and the like.

The subject to be administered with the agent for treating cardiac diseases including the laminate of the present invention is not particularly limited, and preferred examples thereof include human beings, non-human primates (monkey and the like), dogs, cats, pigs, horses, goats, lambs, rats, mice, hamsters, and the like. Among these, human beings are more preferable.

<Film for being laminated on cell sheet>

As described above, by laminating a biocompatible polymer film having a density of 500 $\mu$g/cm$^2$ to 10 mg/cm$^2$ on a cell sheet, the laminate of the present invention can be manufactured. Therefore, according to the present invention, there is provided a film for being laminated on a cell sheet that is formed of a biocompatible polymer film having a density of 500 $\mu$g/cm$^2$ to 10 mg/cm$^2$. Details and preferred aspects of the biocompatible polymer film are as described above in the present specification.

The present invention will be more specifically described based on the following examples, but the present invention is not limited to the examples.

Examples

(1) Recombinant gelatin

As recombinant gelatin, the following CBE3 (described in WO2008/103041A) was prepared.

CBE3:

Molecular weight: 51.6 kD
Structure: GAP[(GXY)$_{63}$]$_3$G
Number of amino acids: 571
Number of RGD sequences: 12
Content of imino acid: 33%

Approximately 100% of the amino acids are repeating GXY structures. The amino acid sequence of CBE3 does not contain serine, threonine, asparagine, tyrosine, and cysteine. CBE3 has an ERGD sequence.

Isoelectric point: 9.34
GRAVY value: -0.682
1/IOB value: 0.323

Amino acid sequence (SEQ ID NO: 1 in sequence listing) (same as SEQ ID NO: 3 in WO2008/103041A except that the terminal X is revised to "P")

GAP(GAPGLQGAPGLQGMPGERGAAGLPGPKGERGDAGPKGADGAPGAPGLQGMPG

ERGAAGLPGPKGERGDAGPKGADGAPGKDGVRGLAGPIGPPGERGAAGLPGPKGER

GDAGPKGADGAPGKDGVRGLAGPIGPPGPAGAPGAPGLQGMPGERGAAGLPGPKGE

RGDAGPKGADGAPGKDGVRGLAGPP)3G

(2) Preparation of recombinant gelatin film

As a typical example of the polymer film, a recombinant gelatin film was prepared using CBE3 of Example 1. Aqueous CBE3 solutions at a concentration of 1% by mass, 2% by mass, 3% by mass, or 4% by mass were prepared, and 4 mL of each of these aqueous CBE3 solutions was poured into a plastic tray in which a silicone frame (8 cm $\times$ 10 cm) was installed. The plastic tray was dried at 4°C or room temperature until no moisture remained, thereby obtaining a recombinant gelatin film.

The recombinant gelatin film was taken out of the plastic tray/silicone frame and subjected to thermal crosslinking at 160°C under reduced pressure (crosslinking was performed 6 hours, 12 hours, or 24 hours), thereby obtaining an insoluble recombinant gelatin film sample.

By the aforementioned preparation method, recombinant gelatin films having a density of 500 $\mu$g/cm$^2$, 1 mg/cm$^2$, 1.5 mg/cm$^2$, and 2.0 mg/cm$^2$ were obtained. The density of the recombinant gelatin films was calculated by "coating mass/coating area" at the time of preparation. The coating mass means the mass of the recombinant gelatin applied.

(3) Evaluation of swelling ratio of polymer film

For each of the recombinant gelatin films prepared in (2) described above, the swelling ratio was evaluated as a value of physical properties. For the evaluation, the recombinant gelatin film was punched using a biopsy punch having a diameter of 8 mm, thereby preparing a disk-like film having a diameter of 8 mm. At this time, the thickness of the dry film was measured and adopted as a dry film thickness. The disk-like film was sufficiently wet with water for injection, and then the thickness of the wet film was measured and adopted as a wet film thickness. The film thickness was measured using a micrometer (SOFT TOUCH MICRO CLM manufactured by Mitutoyo Corporation).

From the wet film thickness and the dry film thickness, a swelling ratio ((swollen film thickness [$\mu$m]/dry film thickness [$\mu$m]) $\times$ 100) was calculated.

(4) Evaluation of handleability of polymer film

By using the wet disk-like film obtained in (3) described above, the handleability was evaluated. One side of the wet disk-like film was held with tweezers, and the film was picked up horizontally. At this time, in case where the film was kept horizontal, the handleability was evaluated A; in a case where the tip of the film (side far from the tweezers) hung down and bent at an angle of equal to or greater than 30°, the handleability was evaluated B; and in a case where the film was folded in half, the handleability was evaluated C. Even a film evaluated as C in terms of the handleability can be put to practical use without a problem by devising how to use it as desired (for example, putting the film in a frame for use, and the like).

The results of the handleability obtained in a case where the polymer film was laminated on a cell sheet were the same as the result of the handleability obtained in a case where only the polymer film was used.

(5) Summary of evaluation of swelling ratio, dry film thickness, and handleability

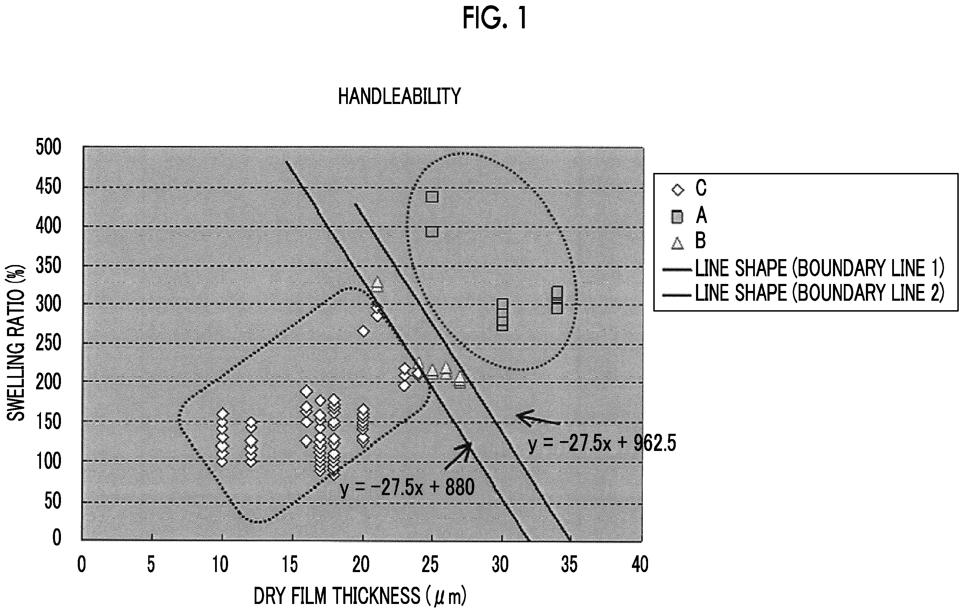

All the results of the evaluation in (3) and (4) described above are summarized in a graph created by plotting the swelling ratio and the dry film thickness on the ordinate and the abscissa respectively (Fig. 1). Furthermore, the points plotted based on the handleability evaluation (A, B, and C) of each of the polymer films are grouped (Fig. 1). As a result, it was understood that in the graph of the dry film thickness and the swelling ratio, boundary lines correlated to the handleability can be drawn.

It was found that in the boundary between A and B of handleability, there is a boundary line (boundary line 2) represented by swelling ratio ((swollen film thickness [$\mu$m]/dry film thickness [$\mu$m]) $\times$ 100) = -27.5 $\times$ dry film thickness [$\mu$m] + 962.5.

It was found that in the boundary between B and C of handleability, there is a boundary line (boundary line 1) represented by swelling ratio ((swollen film thickness [$\mu$m]/dry film thickness [$\mu$m]) $\times$ 100) = -27.5 $\times$ dry film thickness [$\mu$m] + 880.

Therefore, it was understood that regarding the handleability of the polymer film, the polymer film preferably satisfies swelling ratio ((swollen film thickness [$\mu$m]/dry film thickness [$\mu$m]) $\times$ 100) $\geq$ -27.5 $\times$ dry film thickness [$\mu$m] + 880, and more preferably satisfies swelling ratio ((swollen film thickness [$\mu$m]/dry film thickness [$\mu$m]) $\times$ 100) $\geq$ -27.5 $\times$ dry film thickness [$\mu$m] + 962.5.

(6) Protein permeation test for polymer film

By using the polymer films prepared in (2) described above, a protein component permeation test was performed. As a protein component, albumin (molecular weight: 66 kDa) was selected as a typical protein. In a case where this protein component permeates the film, it means that nutrients can be taken through the film.

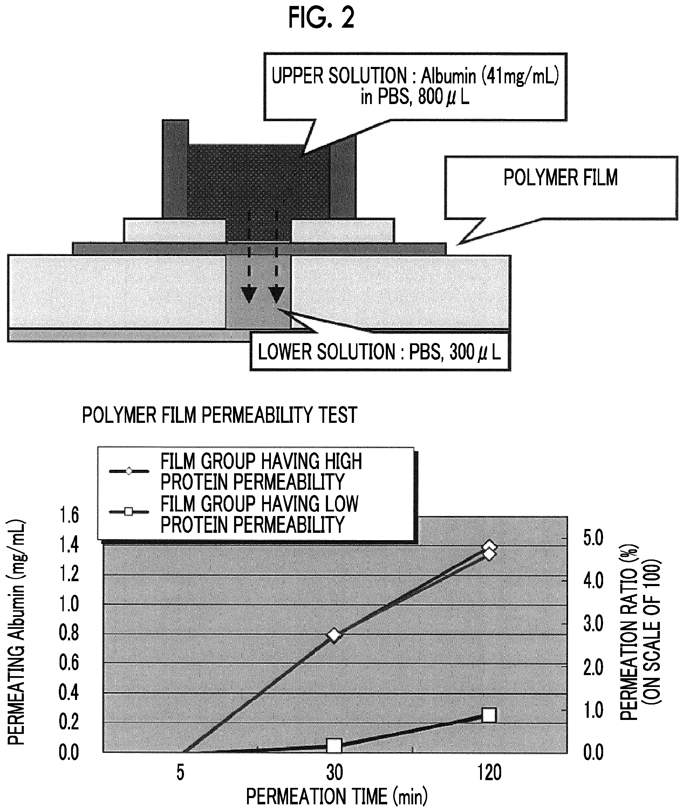

Fig. 2 shows a schematic view of the test system and the results. As an upper solution, an albumin solution obtained by dissolving albumin in phosphate-buffered saline (PBS) at 41 mg/mL was installed. Various polymer films were installed under the upper solution, and the amount of albumin permeating the films and reaching a lower solution (PBS) was measured over time. For measuring the albumin, BIO-RAD PROTEIN ASSAY (manufactured by Bio-Rad Laboratories, Inc.) was used.

As a result, it was understood that albumin molecules permeate the polymer film and reach the lower solution (Fig. 2). Furthermore, in the process of testing various films, it was understood that the films are divided into a film group having high protein permeability (a group) and a film group having low protein permeability (b group).

(7) Protein permeation test for polymer film (sodium dodecyl sulfate-polyacrylamide gel electrophoresis (SDS-PAGE))

Regarding the protein permeation test performed in (6) described above, in order to analyze whether the albumin in the upper solution permeates the polymer film and reaches the lower solution while maintaining the same molecular weight without being decomposed, SDS-PAGE was performed to check the molecular weight of the permeating albumin.

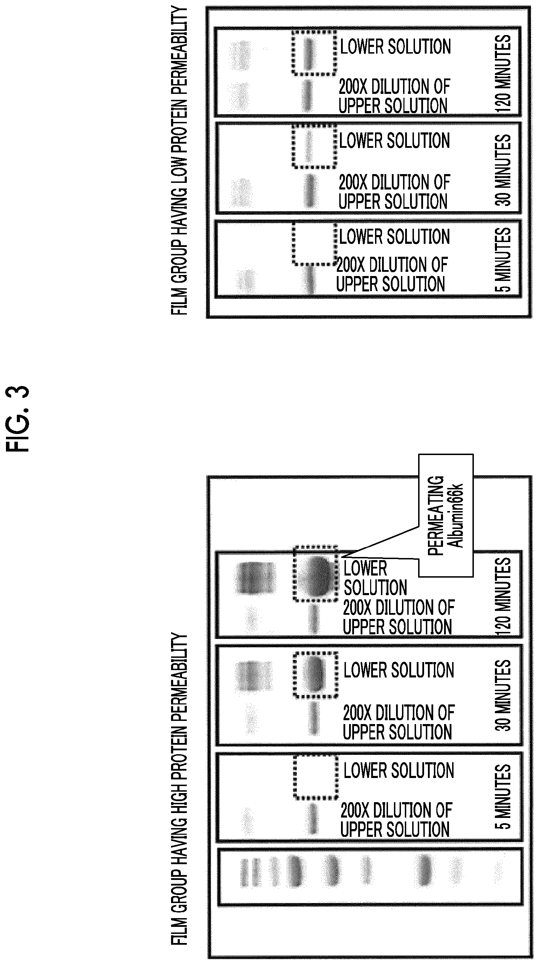

As a result, as shown in Fig. 3, it was understood that for both the film group having high protein permeability and the film group having low protein permeability, the permeating albumin maintains a molecular weight of 66 kDa as in the upper solution. Furthermore, it was confirmed that there is a big difference in the amount of the permeating albumin between the film group having high protein permeability and the film group having low protein permeability.

(8) Protein permeability, swelling ratio, and dry film thickness

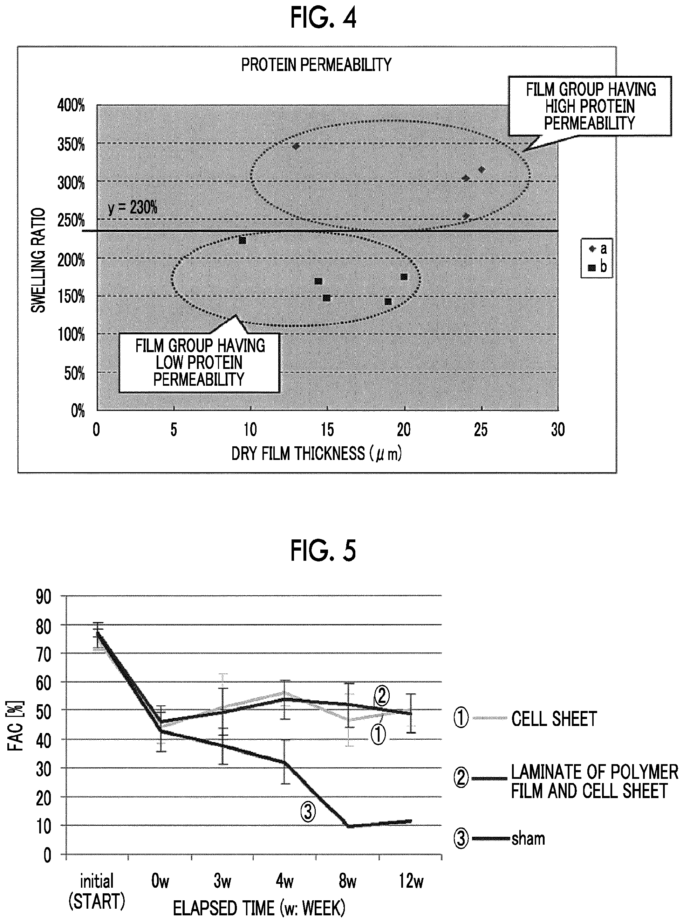

The protein permeability checked in (6) and (7) described above was analyzed by the physical properties of the polymer films. A graph was created by plotting the swelling ratio and the dry film thickness, determined in (3) described above, on the ordinate and the abscissa respectively, and the tested polymer films were plotted on the graph. In addition, the polymer films were divided into a group and b group of (6) and (7) described above (Fig. 4). As a result, it was understood that a boundary represented by swelling ratio = 230 can be drawn as a boundary line between a group and b group.

Accordingly, it was understood that from the viewpoint of protein permeability, by using a polymer film satisfying swelling ratio ≥ 230%, a constitution having high protein permeability can be created. It is considered that in a case where a polymer film satisfying swelling ratio ≥ 230% is used, the polymer film can exert a strong therapeutic effect as a polymer film that the nutrients or humoral factors needed by cells effectively and easily permeate.

(9) Preparation of cell sheet and laminate of recombinant gelatin film and cell sheet

As a typical example of a laminate of a polymer film and a cell sheet, a laminate of a recombinant gelatin film and a cell sheet was prepared. By adding 20% by volume of fetal bovine serum (FBS) (manufactured by MOREGATE BIOTECH) to DMEM low glucose (manufactured by Thermo Fisher Scientific Inc.), a medium was prepared, and rat skeletal myoblasts were suspended in the medium. The cells were seeded in a 48-well temperature-responsive multiwell culture plate (UpCell: manufactured by CellSeed Inc.) at $1.3 \times 10^6$ cells and cultured overnight under the conditions of 37°C and 5% $CO_2$, and then the culture plate was placed at room temperature, thereby obtaining a cell sheet.

A recombinant gelatin film swollen in the same medium was installed on the bottom surface of a silicone frame having a diameter of 6 mm, and rat skeletal myoblasts were seeded at $1.3 \times 10^6$ cells in the same manner as that described above and cultured overnight under the conditions of 37°C and 5% $CO_2$, thereby obtaining a laminate of a recombinant gelatin film and a cell sheet.

(10) Preparation of rat infarction model

8-week-old adult rats (Lewis rats, male, manufactured by Charles River Laboratories Japan, Inc.) were subjected to inhalation anesthesia using isoflurane (manufactured by AbbVie), and tracheal intubation was performed to force the rats to breathe through a small animal ventilator. In this state, the heart was exposed, the coronary artery (LAD) ligation was carried out, and the chest was closed. After 1 week, by using an echocardiographic device (HD11XE, manufactured by Philips Electronics Japan, Ltd.), an image was created along the short axis of the left ventricle at the level of papillary muscle. From the left ventricular end-diastolic area (hereinafter, referred to as LVEDA) and the left ventricular end-systolic area (hereinafter, referred to as LVESA), the cardiac function (fractional area change of the left ventricular cavity, hereinafter, referred to as FAC) was measured (see the following formula). In a case where the value of FAC was lower than 60% in an individual, it was concluded that an infarction model was established, and the rat was used for transplantation.

$$FAC(\%) = \frac{LVEDA - LVESA}{LVEDA} \times 100$$

(11) Transplantation of laminate or cell sheet

The individuals made into infarction models were anesthetized in the same manner as that in the preparation of the model, and their hearts were exposed by intercostal incision. The laminate of the polymer film and the cell sheet or the cell sheet prepared in (9) described above was transplanted to the anterior wall of the left ventricle, and then a fibrin preparation (Chemo-Sero-Therapeutic Research Institute) was applied to the surface thereof. The clotting of the fibrin preparation was checked, and then the chest was closed (for a sham group (placebo group), only the application of the fibrin preparation was performed). The group in which the transplantation of the laminate of the polymer film and the cell sheet was performed consisted of 6 rats, the group in which the transplantation of the cell sheet was performed consisted of 4 rats, and the sham group consisted of 4 rats.

(12) Evaluation of efficacy

The models were observed for 12 weeks after the transplantation of the laminate of the polymer film and the cell sheet or after the transplantation of the cell sheet. Echocardiography was performed before the preparation of the infarction model, at the time of judging the model·transplantation, after 3 weeks from the transplantation, after 4 weeks from the transplantation, after 8 weeks from the transplantation, and after 12 weeks from the transplantation. From the change in FAC, the efficacy was determined. By performing multiple comparison on the groups based on the Tukey-Kramer method, the significant difference in the change in FAC was judged. The results are shown in Fig. 5.

In the group in which the transplantation of the laminate of the polymer film and the cell sheet was performed, the value of FAC was maintained until after the 12th week from the transplantation, and therefore, it was understood that the cardiac function is maintained. In contrast, in the sham group, 3 out of 4 rats died of cardiac failure by after the 12th week from the transplantation, and FAC of the individual surviving until after the 12th week from the transplantation was found to be significantly reduced.

These results show that the transplantation of the laminate of the polymer film and the cell sheet keeps the heart functioning and has an effect of increasing the survival rate. Furthermore, it was confirmed that there is no significant difference in the value of FAC between the group in which the transplantation of the laminate of the polymer film and the cell sheet was performed and the group in which the transplantation of the cell sheet was performed. Accordingly, it is considered that even in a case where the laminate of the polymer film and the cell sheet is transplanted, the therapeutic effect of the cell transplantation will not be further reduced compared to the case where the cell sheet is transplanted.

(13) Pathological analysis

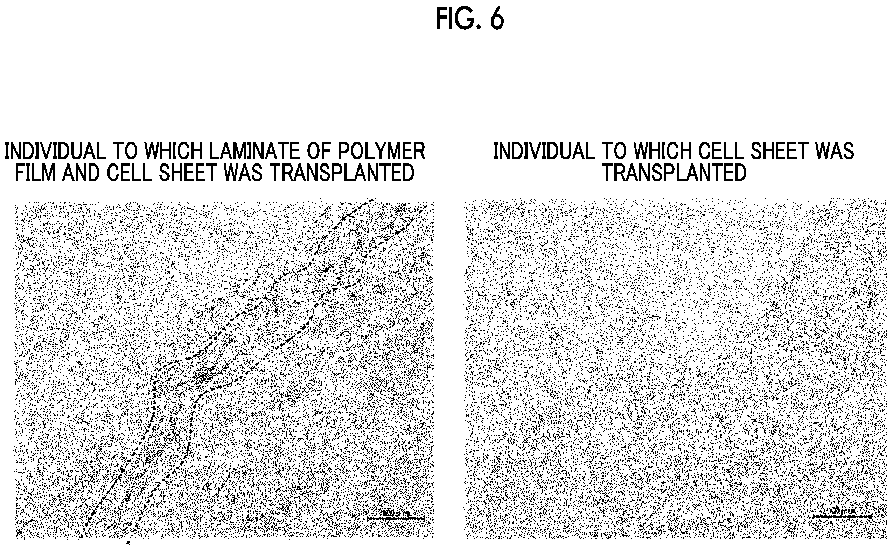

On the 12th week from the transplantation, the heart was fixed using a 10% by mass neutral buffered formalin and sliced in round, and the slices were embedded in paraffin and made into thin sections of 3 to 4 $\mu$m. The sections were deparaffinized, then treated with 0.3% by mass hydrogen peroxide in methanol, and subjected to blocking by using 1% bovine serum albumin (BSA). The sections were mixed with Monoclonal Anti-skeletal Myosin (FAST) Clone MY-32 and Mouse Ascites Fluid (manufactured by Sigma-Aldrich Co. LLC.) and allowed to react overnight under refrigeration. The sections were subjected to visualization by using Envision +Dual Link System-HRP (manufactured by Dako) as secondary antibodies and diaminobenzidine (DAB) and subjected to contrast staining by using hematoxylin. The results are shown in Fig. 6.

As a result, it was confirmed that in the individual to which the laminate of the polymer film and the cell sheet was transplanted, Myosin Heavy Chain-position skeletal myoblasts remained in the form of a layer. These Myosin Heavy Chain-positive cells have a sarcomere structure characteristic of skeletal muscle cells, and are considered to be generated from the skeletal myoblasts that are engrafted and then mature to skeletal muscle cells. In contrast, in the individual to which the cell sheet was transplanted, no such transplanted cells were found to remain. Therefore, it was considered that by transplanting the laminate of the polymer film and the cell sheet so as to keep the cells in an excellent condition after transplantation and to increase the blood flow supplied from the heart including angiogenesis, the engraftment of the transplanted cells can be stimulated.

In addition, on the periphery of the portion to which the laminate of the polymer film and the cell sheet was transplanted, the aggregation of inflammatory monocytes such as lymphocytes was not checked. Therefore, it was considered that the polymer film does not have immunogenicity and can be transplanted to the surface of the heart.

Sequence listing

International application 16F02151 accepted based on Patent Cooperation Treaty Laminate containing cell sheet, Heart JP17012285 20170327----00070284851700658046 Normal 20170327115558201703081040373960_P1AP101_16_1.app

**Claims**

**1.** A laminate comprising:

a biocompatible polymer film having a density of 500 $\mu$g/cm$^2$ to 10 mg/cm$^2$; and
a cell sheet disposed on at least one surface of the biocompatible polymer film,
wherein the cell is a myocardial cell or a skeletal myoblast; and
wherein the biocompatible polymer film satisfies the following Formula 1,

Formula 1: (swollen film thickness/dry film thickness) $\times$ 100 $\geq$ -27.5 $\times$ dry film thickness + 880

where the unit of the swollen film thickness and the dry film thickness is $\mu$m.

**2.** The laminate according to claim 1,
wherein the biocompatible polymer film satisfies the following Formula 2,

Formula 2: (swollen film thickness/dry film thickness) $\times$ 100 $\geq$ -27.5 $\times$ dry film thickness + 962.5

where the unit of the swollen film thickness and the dry film thickness is $\mu$m.

**3.** The laminate according to claim 1 or 2,
wherein a swelling ratio of the biocompatible polymer film represented by the following Formula 3 is equal to or higher than 230%,

Formula 3: (swollen film thickness/dry film thickness) $\times$ 100

where the unit of the swollen film thickness and the dry film thickness is $\mu$m.

**4.** The laminate according to any one of claims 1 to 3,
wherein the dry film thickness of the biocompatible polymer film is 5 to 200 $\mu$m.

**5.** The laminate according to any one of claims 1 to 4,
wherein a wet film thickness of the biocompatible polymer film is 50 to 500 $\mu$m.

**6.** The laminate according to any one of claims 1 to 5,
wherein the biocompatible polymer is recombinant gelatin.

**7.** The laminate according to claim 6,
wherein the recombinant gelatin is represented by the following Formula 4,

Formula 4:        A-[(Gly-X-Y)$_n$]$_m$-B

in the formula, A represents any amino acid or any amino acid sequence, B represents any amino acid or any amino acid sequence, n X's each independently represent any amino acid, n Y's each independently represent any amino acid, n represents an integer of 3 to 100, m represents an integer of 2 to 10, and n sequences represented by Gly-X-Y may be the same as or different from each other.

**8.** The laminate according to claim 6 or 7,
wherein the recombinant gelatin is represented by the following Formula 5,

Formula 5: Gly-Ala-Pro-[(Gly-X-Y)$_{63}$]$_3$-Gly

in the formula, 63 X's each independently represent any amino acid, 63 Y's each independently represent any amino acid, and 63 sequences represented by Gly-X-Y may be the same as or different from each other.

9. The laminate according to any one of claims 6 to 8,
wherein the recombinant gelatin has (1) amino acid sequence described in SEQ ID NO: 1 or (2) amino acid sequence which shares a sequence identity equal to or higher than 80% with the amino acid sequence described in SEQ ID NO: 1 and has biocompatibility.

10. The laminate according to any one of claims 6 to 9,
wherein the recombinant gelatin has the amino acid sequence described in SEQ ID NO: 1.

11. The laminate according to any one of claims 1 to 10 for use in treating cardiac diseases.

**Patentansprüche**

1. Laminat aufweisend:

einen biokompatiblen Polymerfilm mit einer Dichte von 500 $\mu$g/cm$^2$ bis 10 mg/ cm$^2$; und
eine Zellschicht, die sich an mindestens einer Oberfläche des biokompatiblen Polymerfilms befindet,
wobei die Zelle eine Herzmuskelzelle oder ein Skelettmyoblast ist; und
wobei der biokompatible Polymerfilm die folgende Formel 1 erfüllt,

Formel 1: (Filmdicke im gequollenen Zustand/Filmdicke im trockenen Zustand) x 100 ≥ -27,5 x Filmdicke im trockenen Zustand + 880

wobei die Einheit der Filmdicke im gequollenen Zustand und der Filmdicke im trockenen Zustand $\mu$m ist.

2. Laminat nach Anspruch 1,
wobei der biokompatible Polymerfilm die folgende Formel 2 erfüllt,

Formel 2: (Filmdicke im gequollenen Zustand/Filmdicke im trockenen Zustand) x 100 ≥ -27,5 x Filmdicke im trockenen Zustand + 962,5

wobei die Einheit der Filmdicke im geqollenen Zustand und der Filmdicke im trockenen Zustand $\mu$m ist.

3. Laminat nach Anspruch 1 oder 2,
wobei ein Quellverhältnis des biokompatiblen Polymerfilms, das durch die folgende Formel 3 repräsentiert wird, gleich 230% oder höher ist,

Formel 3: (Filmdicke im gequollenen Zustand/Filmdicke im trockenen Zustand) x 100

wobei die Einheit der Filmdicke im gequollenen Zustand und der Filmdicke im trockenen Zustand $\mu$m ist.

4. Laminat nach einem der Ansprüche 1 bis 3,
wobei die Filmdicke des biokompatiblen Polymerfilms im trockenen Zustand 5 bis 200 $\mu$m ist.

5. Laminat nach einem der Ansprüche 1 bis 4,
wobei eine Filmdicke des biokompatiblen Polymerfilms im nassen Zustand 50 bis 500 $\mu$m ist.

6. Laminat nach einem der Ansprüche 1 bis 5,

wobei das biokompatible Polymer rekombinante Gelatine ist.

7. Laminat nach Anspruch 6,
   wobei die rekombinante Gelatine repräsentiert wird durch die folgende Formel 4,

   Formel 4:       A-[(Gly-X-Y)$_n$]$_m$-B

   wobei in der Formel A irgendeine Aminosäure oder irgendeine Aminosäuresequenz repräsentiert, B irgendeine Aminosäure oder irgendeine Aminosäuresequenz repräsentiert, n X's jeweils unabhängig irgendeine Aminosäure repräsentieren,
   n Y's jeweils unabhängig irgendeine Aminosäure repräsentieren, n eine ganze Zahl von 3 bis 100 repräsentiert, m eine ganze Zahl von 2 bis 10 repräsentiert, und n Sequenzen, die durch Gly-X-Y repräsentiert werden, gleich oder verschieden voneinander sein können.

8. Laminat nach Anspruch 6 oder 7,
   wobei die rekombinante Gelatine durch die folgende Formel 5 repräsentiert wird,
   Formel 5: Gly-Ala-Pro-[(Gly-X-Y)$_{63}$]$_3$-Gly
   wobei in der Formel, 63 X's jeweils unabhängig irgendeine Aminosäure repräsentieren, 63 Y's jeweils unabhängig irgendeine Aminosäure repräsentieren, und 63 Sequenzen, die durch Gly-X-Y repräsentiert werden, gleich oder verschieden voneinander sein können.

9. Laminat nach einem der Ansprüche 6 bis 8,
   wobei die rekombinante Gelatine (1) die in SEQ ID NO: 1 beschriebene Aminosäuresequenz oder (2) eine Aminosäuresequenz, die eine Sequenzidentität von 80% oder höher mit der in SEQ ID NO: 1 beschriebenen Aminosäuresequenz hat, besitzt und Biokompatibilität besitzt.

10. Laminat nach einem der Ansprüche 6 bis 9,
    wobei die rekombinante Gelatine die in SEQ ID NO: 1 beschriebene Aminosäuresequenz hat.

11. Laminat nach einem der Ansprüche 1 bis 10 zur Verwendung bei der Behandlung von Herzkrankheiten.


**Revendications**

1. Stratifié, comprenant :

   un film polymère biocompatible présentant une densité de 500 $\mu$g/m$^2$ à 10 mg/cm$^2$, et
   une feuille de cellules disposée sur au moins une surface du film polymère biocompatible,
   dans lequel la cellule est une cellule myocardique ou un myoblaste squelettique, et
   dans lequel le film polymère biocompatible satisfait la formule suivante 1 :

   Formule 1 :

   (épaisseur de film gonflé/épaisseur de film sec) x 100 $\geq$ -27,5 x épaisseur de film sec + 880

   où l'unité pour l'épaisseur de film gonflé et l'épaisseur de film sec est le $\mu$m.

2. Stratifié selon la revendication 1,
   dans lequel le film polymère biocompatible satisfait la formule suivante 2 :

   Formule 2 :

   (épaisseur de film gonflé/épaisseur de film sec) x 100 $\geq$ -27,5 x épaisseur de film sec + 962,5

   où l'unité pour l'épaisseur de film gonflé et l'épaisseur de film sec est le $\mu$m.

**3.** Stratifié selon la revendication 1 ou 2,
dans lequel un taux de gonflement du film polymère biocompatible représenté par la formule suivante 3 est supérieur ou égal à 230 % :

Formule 3 :

(épaisseur de film gonflé/épaisseur de film sec) x 100,

où l'unité pour l'épaisseur de film gonflé et l'épaisseur de film sec est le $\mu$m.

**4.** Stratifié selon l'une quelconque des revendications 1 à 3,
dans lequel l'épaisseur de film sec du film polymère biocompatible s'étend de 5 à 200 $\mu$m).

**5.** Stratifié selon l'une quelconque des revendications 1 à 4,
dans lequel l'épaisseur de film humide du film polymère biocompatible s'étend de 50 à 500 $\mu$m.

**6.** Stratifié selon l'une quelconque des revendications 1 à 5,
dans lequel le polymère biocompatible est de la gélatine recombinée.

**7.** Stratifié selon la revendication 6,
dans lequel la gélatine recombinée est représentée par la formule suivante 4 :

Formule 4 : $A\text{-}[Gly\text{-}X\text{-}Y)_n]_m\text{-}B$

dans la formule, A représente un acide aminé quelconque ou une quelconque séquence d'acides aminés ; B représente un acide aminé quelconque ou une quelconque séquence d'acides aminés ; les n X représentent chacun indépendamment un acide aminé quelconque ; les n Y représentent chacun indépendamment un acide aminé quelconque ; n représente un entier allant de 3 à 100 ; m représente un entier allant de 2 à 10, et les n séquences représentées par Gly-X-Y peuvent être identiques ou différentes entre elles.

**8.** Stratifié selon la revendication 6 ou 7,
dans lequel la gélatine recombinée est représentée par la formule suivante 5 :

Formule 5 : $Gly\text{-}Ala\text{-}Pro\text{-}[(Gly\text{-}X\text{-}Y)_{63}]_3\text{-}Gly$

dans la formule, les 63 X représentent chacun indépendamment un acide aminé quelconque ; les 63 Y représentent chacun indépendamment un acide aminé quelconque ; et les 63 séquences représentées par Gly-X-Y peuvent être identiques ou différentes entre elles.

**9.** Stratifié selon l'une quelconque des revendications 6 à 8,
dans lequel la gélatine recombinée présente (1) une séquence d'acides aminés décrite dans SEQ ID NO : 1, ou (2) une séquence d'acides aminés, laquelle partage une identité de séquence supérieure ou égale à 80 % avec la séquence d'acides aminés décrite dans SEQ ID NO : 1, et présente une biocompatibilité.

**10.** Stratifié selon l'une quelconque des revendications 6 à 9,
dans lequel la gélatine recombinée présente la séquence d'acides aminés décrite dans SEQ ID NO : 1.

**11.** Stratifié selon l'une quelconque des revendications 1 à 10 à utiliser dans le traitement de maladies cardiaques.

# FIG. 1

HANDLEABILITY

EP 3 438 246 B1

# FIG. 2

UPPER SOLUTION : Albumin (41mg/mL)
in PBS, 800 μL

POLYMER FILM

LOWER SOLUTION : PBS, 300 μL

POLYMER FILM PERMEABILITY TEST

FILM GROUP HAVING HIGH
PROTEIN PERMEABILITY

FILM GROUP HAVING LOW
PROTEIN PERMEABILITY

EP 3 438 246 B1

FIG. 3

25

## FIG. 4

## FIG. 5

# FIG. 6

INDIVIDUAL TO WHICH LAMINATE OF POLYMER
FILM AND CELL SHEET WAS TRANSPLANTED

INDIVIDUAL TO WHICH CELL SHEET WAS
TRANSPLANTED

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- JP 2010081829 A
- WO 2008103041 A
- EP 3006559 A1
- WO 2014192909 A1
- EP 2940126 A1
- WO 2014104366 A1
- WO 2006107207 A2
- EP 1014176 A
- US 6992172B A
- WO 200485473 A
- EP 1014176 A2
- US 6992172 B
- JP 17012285 A
- JP 20170327 A

### Non-patent literature cited in the description

- *Pharmaceutical Bulletin,* 1954, vol. 2 (2), 163-173
- *Scope of Chemistry,* 1957, vol. 11 (10), 719-725
- *Fragrance Journal,* 1981, vol. 50, 79-82
- **YOSHIKI KODA et al.** New Edition of Organic Conception Diagram-Fundamentals and Applications. SANKYO SHUPPAN Co., Ltd, 2008
- Protein Identification and Analysis Tools on the ExPASy Server. **GASTEIGER E. ; HOOGLAND C. ; GATTIKER A. ; DUVAUD S. ; WILKINS M.R. ; APPEL R.D. ; BAIROCH A.** The Proteomics Protocols Handbook. Humana Press, 2005, 571-607
- **GASTEIGER E. ; GATTIKER A. ; HOOGLAND C. ; IVANYI I. ; APPEL R.D. ; BAIROCH A.** ExPASy: the proteomics server for in-depth protein knowledge and analysis. *Nucleic Acids Res.,* 2003, vol. 31, 3784-3788
- Pathophysiology. Nagai Publishing Co., Ltd, 1990, vol. 9, 527
- **MAXEY, C. R.** P. J. Academic. 1976
- *J. Mol. Biol.,* 1990, vol. 215, 403-410